# EUROPÄISCHE PATENTSCHRIFT

(11) **EP 4 225 236 B1**
(45) Veröffentlichungstag und Bekanntmachung des Hinweises auf die Patenterteilung: **27.05.2026**
(21) Anmeldenummer: 21790462.2
(22) Anmeldetag: 12.10.2021
(51) Int. Cl.: A61F 9/009, A61F 9/008

(54) **VERFAHREN ZUR ZENTRIERUNG EINES KONTAKTGLASES UND REFRAKTIVES CHIRURGISCHES LASERSYSTEM**
METHOD FOR CENTRING A CONTACT LENS AND REFRACTIVE SURGICAL LASER SYSTEM
PROCÉDÉ DE CENTRAGE D'UNE LENTILLE DE CONTACT ET SYSTÈME LASER DE CHIRURGIE RÉFRACTIVE

(30) Priorität: 12.10.2020 DE 102020212850
(43) Veröffentlichungstag der Anmeldung: 16.08.2023
(73) Patentinhaber: Carl Zeiss Meditec AG, 07745 Jena (DE)
(72) Erfinder: WEYHAUSEN, Andreas, 07745 Jena (DE); BISCHOFF, Mark, 07745 Jena (DE); STOBRAWA, Gregor, 07745 Jena (DE); LEHNORT, Marco, 07745 Jena (DE)
(74) Vertreter: Pearl Cohen EU
(86) Internationale Anmeldenummer: PCT/EP2021/078134
(87) Internationale Veröffentlichungsnummer: WO 2022/078998

(56) Entgegenhaltungen:
- US-A1- 2008 071 254
- US-A1- 2009 163 898
- US-A1- 2011 304 819
- US-A1- 2014 276 678

## Beschreibung

Die vorliegende Erfindung betrifft ein Verfahren zur Zentrierung eines Kontaktglases auf ein Patientenauge, ein Verfahren zur Vorbereitung einer refraktiven chirurgischen Behandlung eines Auges, eine Recheneinheit und ein refraktives chirurgisches Lasersystem. Die Erfindung liegt somit insbesondere auf dem Gebiet der Lasersysteme für die refraktive Chirurgie.

Für refraktive chirurgische Behandlungen des Auges werden oftmals ophthalmologische Femtosekunden-Laser eingesetzt, wobei mit dem Laserstrahl refraktive Augenkorrekturen durch ein Ablösen bzw. Herauslösen von Gewebe aus dem Auge, insbesondere aus der Hornhaut, durchgeführt werden. Zur Einkopplung des Laserstrahls in das zu behandelnde Auge wird typischerweise ein Kontaktglas mit einer Kontaktfläche verwendet, welches auf das zu behandelnde Auge des Patienten aufgesetzt wird und an dem Auge festgesaugt wird, um das Auge zu fixieren. Durch das Kontaktglas hindurch kann sodann der Laserstrahl in das festgesaugte Auge eingekoppelt werden und kontrolliert auf das daran fixierte Auge appliziert werden.

Das Zentrum des Kontaktglases, welches typischerweise durch den Scheitelpunkt der gekrümmten Kontaktglasfläche definiert ist, wird dabei als geometrischer Ursprung aller vom Laser durchgeführten Schnitte im Auge verwendet. Daher bestimmt die Positionierung bzw. Zentrierung des Kontaktglases in Bezug auf das Auge die Lage der Schnitte im Auge. Für die Genauigkeit der refraktiven chirurgischen Behandlung ist daher eine präzise und kontrollierte Positionierung und Zentrierung des Kontaktglases von großer Bedeutung.

Das Positionieren des Kontaktglases umfasst typischerweise die folgenden drei Phasen:
Eine Grobpositionierung, bei welcher sich das Kontaktglas im Abstand von einigen Zentimetern bis Millimetern über dem Auge des Patienten befindet und das Kontaktglas am Auge ausgerichtet wird.

Eine Feinpositionierung, bei welcher das Kontaktglas mit dem Auge in Berührung ist und in seine gewünschte finale Position relativ zum Auge gebracht wird.

Ein angesaugter Zustand, in welchem die Ansaugung (Suction) des Auges an das Kontaktglas bzw. umgekehrt aktiviert ist, sodass das Patientenauge am Kontaktglas fixiert ist.

Herkömmliche Lasersysteme für die refraktive Chirurgie bieten dem Arzt verschiedenartige Unterstützung bei der Grobpositionierung des Kontaktglases relativ zum Auge des Patienten, wie etwa ein Fixationslicht, anhand dessen sich das Auge zum Kontaktglas in Winkelrichtung ausrichten kann. Die Feinpositionierung erfolgt jedoch herkömmlicherweise durch den Arzt, welcher nach eigenem Ermessen und in Abhängigkeit des Behandlungstyps die exakte Positionierung des Kontaktglases am Auge, genauer gesagt den gewünschten Zielpunkt auf der Kornea, festlegt.

Die Feinpositionierung wird jedoch als Herausforderung wahrgenommen, da diese bei vielen Ärzten ein hohes Maß an Verunsicherung hervorruft und durch den Arzt auch mehrere oder gar zahlreiche Positionierungsversuche durchgeführt werden, bis eine für den Arzt zufriedenstellende Positionierung erreicht ist. Dabei erfolgt herkömmlicherweise eine Feinpositionierung bzw. Zentrierung des Kontaktglases beispielsweise derart, dass der Arzt ein ausgedrucktes Diagnosebild des zu behandelnden Auges heranzieht, auf welchem der gewünschte Positionierungspunkt eingezeichnet ist und auf Basis dessen sich der Arzt bei der Zentrierung des Kontaktglases am realen Auge zurechtzufinden versucht. Auch erfolgt herkömmlicherweise keine Protokollierung der festgelegten Feinpositionierung, sodass eine nachträgliche Analyse diesbezüglich nicht möglich ist. Eine quantitative Beurteilung und Protokollierung der erreichten Zentrierung (bzw. Abweichung vom angestrebten Ziel) ist somit nur manuell möglich.

Auch haben Ärzte bei der Feinpositionierung häufig die Absicht, bei angesaugtem Auge nochmals eine Verschiebung des Kontaktglases bzw. des Behandlungszentrums vorzunehmen, was mit den herkömmlichen Systemen nicht möglich ist und daher ein Lösen des Auges vom Kontaktglas und erneutes Fixieren erfordern kann.

Die US 2011/304,819 A1 beschreibt ein Docking Verfahren für ein ophthalmisches Linsensystem an ein Auge, welches das Erfassen eines Bildes einer internen Struktur des Auges umfasst.

Es ist daher die Aufgabe der Erfindung, Verfahren und ein refraktives chirurgisches Lasersystem bereitzustellen, welche für den Benutzer bzw. den Arzt die Feinpositionierung des Kontaktglases am Auge vereinfachen und eine reproduzierbar zuverlässige Feinpositionierung erlauben.

Diese Aufgabe wird erfindungsgemäß gelöst durch Verfahren, eine Recheneinheit und ein refraktives chirurgisches Lasersystem mit den Merkmalen der jeweiligen unabhängigen Ansprüche. Vorteilhafte Ausgestaltungen sind in den Unteransprüchen und in der Beschreibung angegeben.

In einem ersten Aspekt betrifft die Erfindung ein Verfahren zur Zentrierung eines Kontaktglases (16) relativ zu einem Auge (12) eines Patienten. Das Verfahren umfasst a) ein Bereitstellen eines Fixierlichts (20) durch das Kontaktglas (16), sodass das Auge (12) des Patienten durch Fixieren auf das Fixierlicht (20) relativ zum Kontaktglas (16) ausgerichtet wird. Ferner umfasst das Verfahren b) ein Erfassen einer Abbildung eines durch eine Lichtquelle mit fester Ortsbeziehung relativ zum Kontaktglas bereitgestellten Lichtmusters (24), wobei die Abbildung des Lichtmusters (24) über eine Reflexion an der Oberfläche des Auges (12) erfolgt. Außerdem umfasst das Verfahren c) ein Darstellen der Abbildung des Lichtmusters (24) über das Auge (12) durch das Kontaktglas (16) in Überlagerung mit virtuellen Markierungen (26), wobei eine erste Markierung der virtuellen Markierungen (26, 26a, 26b) die Zentralachse (200) des Kontaktglases (16) kenntlich macht und eine zweite Markierung der virtuellen Markierungen (26, 26a, 26b) eine Referenzmarkierung kenntlich macht, welche aus der Abbildung des Lichtmusters als auf der Zentralachse des Kontaktglases (16) liegend abgeleitet ist. Zudem umfasst das Verfahren d) ein laterales Positionieren des Kontaktglases (16) relativ zum Auge (12) derart, dass ein Abstand zwischen der ersten und der zweiten Markierung minimiert wird, sowie e) ein Festlegen der Stelle des Auges (12), an welcher sich die zweite Markierung befindet, wenn die erste Markierung und die zweite Markierung den minimierten Abstand zueinander einnehmen, als Position des Vertex (22) des Auges (12) und Registrieren der Position des Vertex (22) anhand eines in der Abbildung des Auges (12) erkennbaren Merkmals des Auges (12).

In einem weiteren Aspekt betrifft die Erfindung ein Verfahren zur Vorbereitung einer refraktiven chirurgischen Behandlung eines Auges mittels eines Lasersystems. Das Verfahren umfasst ein erfindungsgemäßes Verfahren zur Zentrierung eines Kontaktglases relativ zum Auge und ein Ermitteln einer Überdeckung der Pupille des Auges durch eine optische Zone eines bei der refraktiven chirurgischen Behandlung herauszulösenden Lentikels.

In einem weiteren Aspekt betrifft die Erfindung eine Recheneinheit, welche dazu eingerichtet ist, ein refraktives chirurgisches Lasersystem zur Durchführung eines erfindungsgemäßen Verfahrens anzusteuern.

In einem weiteren Aspekt betrifft die Erfindung ein refraktives chirurgisches Lasersystem, vorzugsweise ein Femtosekunden-Lasersystem, mit einem Kontaktglas. Das Lasersystem ist dazu eingerichtet, a) ein Fixierlicht durch das Kontaktglas derart bereitzustellen, dass das Auge eines Patienten durch Fixieren auf das Fixierlicht relativ zum Kontaktglas ausgerichtet wird. Ferner ist das Lasersystem dazu eingerichtet, b) eine Abbildung eines durch eine Lichtquelle mit fester Ortsbeziehung relativ zum Kontaktglas bereitgestellten Lichtmusters zu erfassen, wobei die Abbildung des Lichtmusters über eine Reflexion an der Oberfläche des Auges erfolgt. Zudem ist das Lasersystem dazu eingerichtet, c) eine Abbildung des Lichtmusters über die Reflexion an der Oberfläche des Auges durch das Kontaktglas in Überlagerung mit virtuellen Markierungen darzustellen, wobei eine erste Markierung der virtuellen Markierungen die Zentralachse des Kontaktglases kenntlich macht und eine zweite Markierung der virtuellen Markierungen eine Referenzmarkierung kenntlich macht, welche aus der Abbildung des Lichtmusters als auf der Zentralachse des Kontaktglases liegend abgeleitet ist. Außerdem ist das Lasersystem dazu eingerichtet, d) das Kontaktglas relativ zum Auge derart lateral zu positionieren, dass ein Abstand zwischen der ersten Markierung und der zweiten Markierung minimiert wird, und e) die Stelle des Auges, an welcher sich die zweite Markierung befindet, wenn die erste Markierung und die zweite Markierung den minimierten Abstand einnehmen, als Position des Vertex des Auges festzulegen und die Position des Vertex anhand eines in der Abbildung des Auges erkennbaren Merkmals des Auges zu registrieren.

Dass das Fixierlicht durch das Kontaktglas bereitgestellt wird, bedeutet dabei, dass das Kontaktglas das Fixierlicht selbst bereitstellt, beispielsweise mittels einer entsprechenden in das Kontaktglas integrierten Lichtquelle, oder dass das Fixierlicht von einer separaten Lichtquelle bereitgestellt wird und durch das Kontaktglas hindurch zum Auge transmittiert wird. Das Fixierlicht ist dabei ein für das Auge sichtbares Lichtsignal, welches dem Auge des Patienten zur Orientierung angeboten wird, sodass das Auge die gewünschte Orientierung einnimmt, wenn das Auge des Patienten auf das Fixierlicht fixiert ist. Gemäß einer optionalen Ausführungsform kann die Lichtquelle mit der festen Ortsbeziehung relativ zum Kontaktglas auch als Fixierlicht dienen. Gemäß anderen Ausführungsformen können eine oder mehrere weitere Lichtquellen als Fixierlicht bereitgestellt werden.

"Zentrieren des Kontaktglases relativ zum Auge" bedeutet dabei, dass das Kontaktglas in die gewünschte Position gebracht wird, in welcher die refraktive chirurgische Behandlung am Auge durchgeführt wird. Das Zentrieren erfordert dabei nicht zwingend, dass die Zentralachse des Kontaktglases mit der optischen Achse des Auges zusammenfällt. Vielmehr kann das Kontaktglas auch auf eine andere für die chirurgische Behandlung gewünschte Position des Auges zentriert werden.

Das Darstellen der Abbildung des Auges und der virtuellen Markierungen bedeutet dabei, dass diese für den Benutzer sichtbar gemacht werden, beispielsweise mittels einer Anzeigeeinheit, wie etwa eines Computerdisplays oder eines anderen Anzeigegeräts. Dass die virtuellen Markierungen virtuell sind, bedeutet dabei, dass diese optional nur in der Darstellung angezeigt werden, beispielsweise auf dem Computerdisplay, aber nicht auf das Auge projiziert oder anderweitig am Auge angebracht werden. Gemäß anderen optionalen Ausführungsformen können die virtuellen Markierungen jedoch auch auf das Auge projiziert werden.

Dass die Lichtquelle eine feste Ortsbeziehung zum Kontaktglas aufweist bedeutet dabei, dass sich aus der Position der Lichtquelle die Position des Kontaktglases und optional die Position der Zentralachse des Kontaktglases eindeutig bestimmen lassen. Optional ist die Lichtquelle dazu direkt in und/oder am Kontaktglas angeordnet. Gemäß einer optionalen Ausführungsform ist die Lichtquelle zumindest teilweise ringförmig ausgebildet und umgibt das Kontaktglas zumindest teilweise in Umfangsrichtung. Das Lichtmuster einer solchen Lichtquelle kann in Form eines Lichtrings vorliegen, wobei optional der Mittelpunkt des Lichtrings auf der Zentralachse des Kontaktglases liegt.

Das Lichtmuster ist dabei eine geometrische Anordnung von Licht, welche in der Abbildung erkennbar ist. Das Lichtmuster ist dabei derart ausgestaltet, dass ein ausgezeichneter Punkt des Lichtmusters erfasst werden kann, wie etwa ein Mittelpunkt und/oder ein geometrischer Schwerpunkt und/oder ein Eckpunkt und/oder ein anderer eindeutig festlegbarer Punkt in dem Lichtmuster, anhand dessen sodann die relative Lage der Zentralachse des Kontaktglases ermittelt werden kann.

Dass die Abbildung des Lichtmusters über eine Reflexion an der Oberfläche des Auges erfolgt, bedeutet dabei, dass der Strahlengang des Lichts bei der optischen Abbildung an der Oberfläche des Auges gefaltet wird. Dabei wirkt die gekrümmte Oberfläche des Auges optional als abbildendes optisches Element nach Art eines konvexen Spiegels.

Die Erfindung bietet den Vorteil, dass einem Arzt eine Hilfestellung bei der Zentrierung des Kontaktglases geboten wird, welche eine zuverlässige Zentrierung des Kontaktglases ermöglicht. Insbesondere bietet die Erfindung den Vorteil, dass eine zuverlässige Zentrierung standardmäßig erfolgen kann und auch von solchen Benutzern durchgeführt werden kann, welche nicht über langjährige Erfahrung und Expertise verfügen.

Außerdem bietet die Erfindung den Vorteil, dass eine halbautomatisierte oder vollautomatisierte Zentrierung des Kontaktglases durch das Lasersystem erfolgen kann. Dies bietet entsprechend den Vorteil, dass die vom Benutzer manuell zu erbringenden Tätigkeiten beim Zentrieren reduziert, minimiert oder gar gänzlich eliminiert werden. Dadurch können die Anforderungen an die erforderlichen Kenntnisse und oder motorischen Fähigkeiten des Benutzers reduziert werden. Außerdem kann dem Benutzer die Tätigkeit und insbesondere die Bedienung des Lasersystems erleichtert werden. Zudem kann durch die Erfindung das Risiko von durch den Benutzer verursachten Fehlern reduziert werden.

Auch bietet die Erfindung den Vorteil, dass die Zentrierung des Kontaktglases zuverlässig protokolliert werden kann, und insbesondere die Protokollierung automatisiert erfolgen kann. Dadurch wird die Zuverlässigkeit und die Nachprüfbarkeit der refraktiven chirurgischen Behandlungen erhöht.

Typischerweise ist das beschriebene Fixierlicht senkrecht zur optischen Achse der Behandlungsoptik ausgerichtet. Optional kann das Fixierlicht andere Winkel zur optischen Achse haben, was es für den Patienten als beweglich erscheinen lässt. Optional kann das Fixierlicht abschaltbar sein. Beides ermöglicht dem Arzt bzw. Benutzer, zu überprüfen, ob der Patient mit dem Auge das Fixierlicht tatsächlich fixiert, indem der Arzt bzw. Benutzer das Fixierlicht ein- und ausschaltet und/oder bewegt und die Reaktion des Auges beobachtet.

Optional wird der Mittelpunkt der Pupille des Auges als das in der Abbildung des Auges erkennbare Merkmal des Auges herangezogen. Dies bietet den Vorteil, dass dieser zuverlässig in der Abbildung des Auges ermittelt werden kann, und die Ermittlung zudem automatisiert mittels Bildauswertung erfolgen kann. Optional umfassen die virtuellen Markierungen ferner eine virtuelle Markierung des Mittelpunkts der Pupille. Dies bietet den Vorteil, dass die Position des Mittelpunkts der Pupille auch für den Benutzer einfach erkennbar ist, und der Benutzer diesen anhand der virtuellen Markierung zu Orientierungszwecken verwenden kann. Optional kann eine Infrarot-Beleuchtung des Auges verwendet werden, um eine zuverlässige Erkennung der Pupille und insbesondere des Mittelpunkts der Pupille auch bei dunklen Augen bzw. bei Augen mit einer dunkelfarbigen Iris zu erleichtern.

Optional ist das Lichtmuster ein Ringlicht, welches durch eine als Ringbeleuchtung ausgestaltete Lichtquelle bereitgestellt wird und durch das Kontaktglas hindurch auf das Auge gerichtet ist. Alternativ kann die Ringbeleuchtung auch außerhalb des Kontaktglases liegen. Vorzugsweise ist der Ring konzentrisch um die Zentralachse des Kontaktglases angeordnet. Die Reflexion der Ringbeleuchtung am Auge ist in der Darstellung der Abbildung des Auges erkennbar. Die Verwendung des Projektionsrings bietet den Vorteil, dass bei Reflexion auf der gekrümmten Oberfläche des Auges die Form der Reflexion des Projektionsrings geändert wird, wenn der Projektionsring nicht senkrecht zum und konzentrisch auf den dem Kontaktglas zugewandten Scheitelpunkt des Auges einfällt. Anhand der Formabweichungen der Reflexion des Projektionsrings können sodann optional Rückschlüsse auf die Positionierung des Kontaktglases relativ zum Auge gezogen werden.

Optional bildet der Mittelpunkt des Projektionsrings den Mittelpunkt des Lichtmusters. Sofern das Lichtmuster noch weitere Elemente aufweist, sind diese vorzugsweise konzentrisch um den Mittelpunkt angeordnet. Dies erleichtert das Erkennen der relativen Positionierung des Kontaktglases relativ zum Auge anhand der Verformung der Reflexion der projizierten Markierung. Optional kann das Lichtmuster mehrere Projektionsringe aufweisen, welche optional konzentrisch zur Zentralachse des Kontaktglases angeordnet sind. Alternativ oder zusätzlich kann das Lichtmuster ein vorbestimmtes Punktmuster aufweisen.

Gemäß einer optionalen Ausführungsform weist das Lichtmuster ein Vieleck und/oder ein Gitter und/oder ein Kreuz auf. Das Vieleck kann optional als Dreieck, Viereck, Sechseck oder Achteck ausgebildet sein, wobei auch eine andere Anzahl von Ecken möglich ist. Das Lichtmuster ist dabei optional derart ausgebildet, dass aus einem Mittelpunkt und/oder aus einem Schwerpunkt des Lichtmusters und/oder aus einem anderen ausgezeichneten Punkt die Referenzmarkierung ableitbar ist, welche aus der Abbildung des Lichtmusters als auf der Zentralachse des Kontaktglases liegend abgeleitet ist. Gemäß einer optionalen Ausführungsform ist das Lichtmuster derart ausgebildet, dass anhand von Verzerrungen in der Abbildung des Lichtmusters über die Oberfläche des Auges relativ zu einer Abbildung einer idealen Oberfläche eines Auges die Form der Oberfläche des Auges zumindest teilweise bestimmbar ist.

Optional erfolgt das Erfassen der Abbildung des Lichtmusters über die Reflexion an der Oberfläche des Auges derart, dass das Lichtmuster und das Merkmal des Auges in der Abbildung erkennbar sind. Dies kann beispielsweise durch eine Abbildung mit einer ausreichend großen Schärfentiefe erreicht werden, sodass sowohl die Lichtquelle als auch die Oberfläche des Auges und optional die darunter liegende Iris und Pupille in der erfassten Abbildung scharf abgebildet sind. Dies bietet den Vorteil, dass das Merkmal des Auges, welches zur Registrierung der Position des Vertex herangezogen wird, in derselben Abbildung erkennbar ist, die auch die Lichtquelle bzw. das Lichtmuster abbildet. Optional erfolgt die Abbildung mit einer Schärfentiefe von mindestens 10 mm, optional mindestens 15 mm, optional mindestens 20 mm und optional mindestens 30 mm. Dies bietet den Vorteil, dass sowohl die Ebene der Lichtquelle als auch die Ebene des Merkmals des Auges im Schärfentiefenbereich erfasst werden können.

Optional ist bei der Durchführung der Schritte a) bis e) das Kontaktglas vom Auge beabstandet, wobei optional der Abstand des Kontaktglases zum Auge im Bereich von 1 mm bis 10 cm liegt. Dies bietet den Vorteil, dass die Zentrierung des Kontaktglases erfolgen kann, wenn das Kontaktglas noch nicht in Kontakt mit dem Auge ist. Außerdem bietet dies den Vorteil, dass der durch das Kontaktglas abbildbare Bereich des Auges an die Bedürfnisse angepasst werden kann. Zudem bietet dies den Vorteil, dass die Reflexion der projizierten Markierung zumindest teilweise durch das Kontaktglas transmittiert, detektiert und dargestellt werden kann.

Optional umfasst das Verfahren ferner (f) ein Reduzieren des Abstands zwischen Kontaktglas und Auge und ein Kontaktieren des Auges mit dem Kontaktglas und (g) ein Feinpositionieren des Kontaktglases in lateraler Richtung relativ zum Auge derart, dass die Zentralachse des Kontaktglases an einer vorbestimmten Position des Auges positioniert ist, wobei die Schritte f) und g) optional in beliebiger Reihenfolge und/oder mehrfach durchführbar sind. Dies bietet den Vorteil, dass eine weitere Feinpositionierung dann erfolgen kann, wenn das Kontaktglas bereits in Kontakt mit dem Auge ist. Dies kann insbesondere dahingehend vorteilhaft sein, dass damit Veränderungen ausgeglichen werden können, die sich durch das In-Kontakt-Bringen des Kontaktglases mit dem Auge ergeben, wie etwa Verformungen des Auges und insbesondere der Hornhaut. Auch kann eine Feinpositionierung mehrere Durchläufe in Kontakt mit dem Auge und beabstandet vom Auge umfassen und ermöglicht somit eine schrittweise Heranführung an die gewünschte Position und/oder ein Austesten verschiedener Positionen.

Optional umfasst das Verfahren ferner ein Bestimmen eines Kappa-Winkels anhand der Position des Vertex und des Mittelpunkts der Pupille. Dies bietet den Vorteil, dass insbesondere besonders groß ausgeprägte Kappa-Winkel erkannt werden können, welche typischerweise zu einem unerwünschten Wegdrehen des Auges beim Ansaugen an das Kontaktglas führen. Sofern ein großer Kappa-Winkel ermittelt wurde, kann optional ein Hinweis oder eine Warnung an den Benutzer ausgegeben werden, welcher auf das Vorliegen eines großen Kappa-Winkels und die damit einhergehenden Risiken hinweist. Auch kann optional ein Lasersystem dazu eingerichtet sein, eine Ansaugung des Auges zu unterbinden, wenn ein Kappa-Winkel ermittelt wird, der um ein vorbestimmtes Maß von einer Vorgabe abweicht.

Optional umfassen die virtuellen Markierungen ferner eine virtuelle Markierung eines vorbestimmten Punktes des Auges, wobei der vorbestimmte Punkt des Auges vom Benutzer vorbestimmt ist. Beispielsweise kann der Benutzer einen oder mehrere Punkte am Auge bestimmen, welche ebenfalls mittels einer virtuellen Markierung in der Darstellung der Abbildung des Auges markiert werden. Beispielsweise kann der Benutzer jenen Punkt mit einer virtuellen Markierung versehen, an welchem er die Zentrierung des Kontaktglases vorsieht. Mit anderen Worten kann der Benutzer ein oder mehrere (alternative) benutzerdefinierte Positionierungsziele vorgeben, welche beispielsweise vom Arzt bei der Planung der Behandlung definiert werden können, und optional auch das bzw. die benutzerdefinierten Positionierungsziele jeweils mittels einer virtuellen Markierung in der Darstellung der Abbildung des Auges darstellen lassen. Optional kann der Benutzer die virtuellen Markierungen wahlweise ein- und ausblenden. Beispielsweise kann der Benutzer dazu die Koordinaten des zu markierenden Punktes bereitstellen. Die Bereitstellung der Koordinaten dieses Punktes kann beispielsweise direkt (mittels kartesischer und/oder Radialkoordinaten) in Bezug auf das Pupillenzentrum bzw. den Pupillenmittelpunkt (insbesondere bei einer bestimmten Pupillengröße, zur Berücksichtigung eines Pupil-Center-Shifts) oder auf einer Topografie oder einem Wellenfrontbild oder einem OCT-generierten Bild (z.B. Pachy-Map, Epithelial-Map) des Auges erfolgen.

Optional umfasst der oben erläuterte Schritt (c) des Verfahrens ferner zumindest teilweise ein Anzeigen einer Topografie und/oder eines Wellenfrontbildes und/oder eines OCT-generierten Bildes des Auges, wie etwa einer Pachy-Map und/oder einer Epithelial Map). Optional kann der Benutzer einen oder mehrere der genannten Elemente wahlweise ein- oder ausblenden. Optional kann der Benutzer eine Darstellung einer oder mehrerer derartiger Bildinformationen als teiltransparentes Overlay wählen und/oder zwischen den mehreren Darstellungen der Bildinformationen umschalten.

Optional umfasst die Erfindung ferner ein Erfassen einer am Auge erzeugten Reflexion der projizierten optischen Markierung und ein Charakterisieren einer Form des Auges anhand der erfassten Reflexion des Lichtmusters. Dies bietet die Möglichkeit, mittels Bildauswertung die Position des Kontaktglases relativ zum Auge zumindest teilweise automatisiert zu bestimmen.

Optional werden die Schritte d) und/oder f) und/oder g) vom Benutzer durchgeführt, d.h. nach Wunsch und unter Führung des Benutzers manuell oder vorwiegend manuell. Alternativ oder zusätzlich kann das Verfahren halbautomatisiert oder vollautomatisiert durchführbar sein. Dies bietet den Vorteil, dass die erforderlichen Kenntnisse des Benutzers reduziert werden können und zudem die vom Benutzer zu leistende Arbeit erleichtert werden kann. Sowohl bei teil- als auch bei vollautomatisierter Durchführung kann der Benutzer optional stets manuell eingreifen, um die automatisierten Bewegungen zu unterbrechen, ggf. fortzusetzen oder auch abzubrechen und manuell fortzufahren. Dies bietet den Vorteil, dass beispielweise bei einer Fehlfunktion ein Eingreifen des Benutzers ermöglicht wird.

Optional umfasst das Verfahren ein Überprüfen einer Überdeckung der Pupille durch die optische Zone des Lentikels, optional einschließlich einer Sicherheitsmarge. Hierfür wird bevorzugt die mesopische Pupille verwendet, ggf. auch die skotopische Pupille. Dies bietet den Vorteil, dass das Risiko einer fehlerhaften refraktiven chirurgischen Behandlung reduziert werden kann, indem eine unzureichende Überdeckung bereits vorab erkannt und der Benutzer darauf hingewiesen werden kann.

Optional umfasst das Verfahren ferner das Durchführen einer Plausibilitätsprüfung des ermittelten Abstands zwischen der Position des Vertex und des Mittelpunkts der Pupille. Solch ein Plausibilitätscheck kann beispielsweise auf Basis eines Vergleichs der gemäß den oben bezeichneten Verfahrensschritten ermittelten relativen Lage und somit auch des Abstands des Vertex zum Mittelpunkt der Pupille mit Informationen erfolgen, welche separat mittels anderweitiger Diagnosegeräte ermittelt werden, wie etwa mittels (Scheimpflug-) Topographen und/oder OCTs.

Die Abbildung des Auges durch das Kontaktglas hindurch kann optional mittels einer digitalen Videokamera erfasst werden. Diese kann beispielsweise auf der dem Auge abgewandten Seite des Kontaktglases angeordnet sein und das durch das Kontaktglas hindurch transmittierte Licht zumindest teilweise erfassen und detektieren. Gemäß anderen Ausführungsformen kann auch eine analoge Videokamera verwendet werden.

Optional weist das refraktive chirurgische Lasersystem und insbesondere die digitale Videokamera eine telezentrische Optik auf, welche die Maßstababhängigkeit vom Abstand des Kontaktglases zum Auge eliminiert oder zumindest reduziert. Dies ermöglicht eine zuverlässige Erfassung der Abbildung des Auges mit der Videokamera bei unterschiedlichen Abständen zwischen dem Kontaktglas und dem Auge.

Die Recheneinheit kann dabei optional in das Lasersystem integriert sein oder separat vom Lasersystem ausgebildet sein. Die Recheneinheit kann beispielsweise einen Mikrocontroller und/oder eine CPU und/oder einen Personal Computer aufweisen oder als solcher ausgebildet sein. Die Recheneinheit ist optional dazu eingerichtet, das Lasersystem zumindest teilweise zu steuern und optional eine digitale Videokamera des Lasersystems zu steuern, um die erfasste Abbildung des Auges auf einem Anzeigegerät darzustellen und optional virtuelle Markierungen einzublenden. Die Recheneinheit kann optional noch weitere Funktionen innehaben.

Die oben genannten und im Folgenden erläuterten Merkmale und Ausführungsformen sind dabei nicht nur als in den jeweils explizit genannten Kombinationen offenbart anzusehen, sondern sind auch in anderen technisch sinnhaften Kombinationen und Ausführungsformen vom Offenbarungsgehalt umfasst.

Weitere Einzelheiten und Vorteile der Erfindung sollen nun anhand der folgenden Beispiele und bevorzugten Ausführungsformen mit Bezug auf die Figuren näher erläutert werden.

Es zeigen:
- Fig. 1: ein refraktives chirurgisches Lasersystem gemäß einer optionalen Ausführungsform in einer schematischen Darstellung;
- Fig. 2: eine schematische Erläuterung der kennzeichnenden Parameter des Auges und des Kontaktglases;
- Fig. 3 - 5: bildliche Erläuterungen eines Verfahrens zur Zentrierung eines Kontaktglases gemäß einer optionalen Ausführungsform.

In den folgenden Figuren werden gleiche oder ähnliche Elemente in den verschiedenen Ausführungsformen der Einfachheit halber mit gleichen Bezugszeichen bezeichnet.

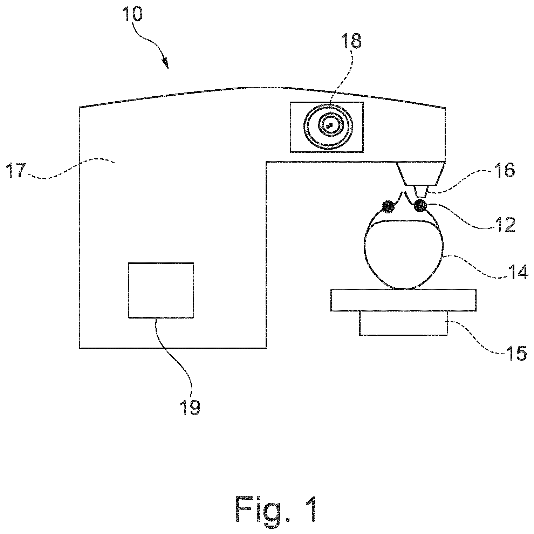

Fig. 1 zeigt in einer schematischen Darstellung ein refraktives chirurgisches Lasersystem 10 zur Durchführung von refraktiven chirurgischen Behandlungen eines Auges 12 eines Patienten 14 gemäß einer optionalen Ausführungsform.

Das Lasersystem 10 weist dabei ein Kontaktglas 16 auf, mittels welchem das Lasersystem 10 an das Auge 12 des Patienten 14 koppelt. Dazu ist der Patient 14 liegend auf einer Liege 15 angeordnet, sodass sein Blick nach oben gerichtet ist und das Lasersystem 10 das Auge mittels des Kontaktglases 16 vertikal von oben kontaktieren und fixieren kann.

Außerdem weist das Lasersystem 10 einen Femtosekunden-Laser 17 auf, welcher in das Lasersystem 10 integriert ist. Der durch den Femtosekunden-Laser 17 bereitgestellte Laserstrahl dient dabei zur refraktiven chirurgischen Behandlung des Auges 12 des Patienten 14 und kann durch das Kontaktglas 16 hindurch auf das Auge 12 appliziert werden.

Ferner weist das Lasersystem 10 eine Anzeigeeinheit 18 auf, mittel welcher dem Benutzer des Lasersystems 10 bzw. dem Arzt eine Abbildung des zu behandelnden Auges 12 des Patienten 14 sowie eine Abbildung einer am Kontaktglas 16 angeordneten Lichtquelle 23 über eine Reflexion an der Oberfläche des Auges 12 dargestellt werden kann. Die darzustellende Abbildung des Auges 12 erfolgt dabei durch das Kontaktglas 16 hindurch, beispielsweise mittels einer digitalen Videokamera (nicht gezeigt), welche in das Lasersystem 10 integriert ist. Die von der digitalen Videokamera erfasste Abbildung des Auges kann sodann zusammen mit überlagerten virtuellen Markierungen durch die Anzeigeeinheit 18 ausgegeben werden, sodass der Arzt bzw. Benutzer des Lasersystems 10 das zu behandelnde Auge 12 und insbesondere dessen Positionierung relativ zum Kontaktglas 16 überprüfen kann.

Das Lasersystem 10 ist dabei derart ausgestaltet, dass eine Relativbewegung des Patienten 14 zum Kontaktglas und/oder zum Lasersystem 10 herbeigeführt werden kann. Dazu kann beispielsweise das Kontaktglas lateral bewegt werden, also senkrecht zur optischen Achse des Kontaktglases 16, um eine geeignete Positionierung des Kontaktglases für die refraktive chirurgische Behandlung des Auges 12 einzunehmen, und auch in longitudinaler Richtung, also entlang der optischen Achse des Kontaktglases, um den Abstand zwischen Kontaktglas 16 und Auge 12 zu ändern und insbesondere um das Kontaktglas 16 am Auge 12 zu fixieren und vom Auge 12 zu lösen. Alternativ oder zusätzlich kann der Patient mittels der Liege 15 vertikal bewegt werden.

Außerdem umfasst das Lasersystem 10 eine Recheneinheit 19, welche dazu eingerichtet ist, das Lasersystem 10 zu steuern und die Abbildung des Auges 12 auf der Anzeigeeinheit 18 anzuzeigen.

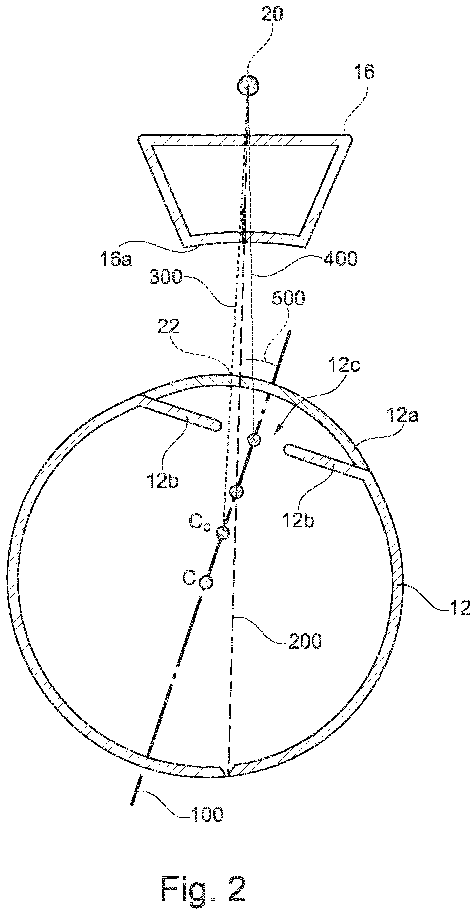

Figur 2 erläutert anhand einer schematischen Darstellung die geometrischen Parameter des Kontaktglases 16 und des Auges 12. In der gezeigten Darstellung ist das Kontaktglas 16 über dem Auge 12 und vom Auge 12 beabstandet positioniert. An der dem Auge 12 zugewandten Seite weist das Kontaktglas 16 eine gekrümmte Kontaktfläche 16a auf, deren Krümmungsradius in etwa dem Krümmungsradius der Hornhaut 12a des Auges 12 entspricht. Das Kontaktglas 16 ist dabei transparent für die Wellenlänge des Laserstrahls und optional auch für den sichtbaren Spektralbereich, um die Beobachtung des Auges durch das Kontaktglas 16 hindurch zu ermöglichen. Auf der dem Auge 12 abgewandten Seite des Kontaktglases 16 befindet sich die Punktlichtquelle für ein Fixierlicht 20, welches sich entlang einer Zentralachse bzw. visuellen Achse 200 des Kontaktglases 16 durch das Kontaktglas zum Auge 12 hin ausbreitet. Die Begriffe Zentralachse 200 des Kontaktglases 16 und visuelle Achse 200 des Kontaktglases 16 werden als Synonyme verwendet.

Das Auge 12 ist in der gezeigten Darstellung derart positioniert und orientiert, dass die optische Achse 100 des Auges 12 schräg nach rechts oben verläuft. Die optische Achse 100 verläuft durch den Mittelpunkt C des Auges und mittig durch die Pupille 12c, welche durch die Iris 12b begrenzt wird.

Auf der optischen Achse 100 des Auges liegt ferner das Krümmungszentrum der Kornea, welches als C_{c} bezeichnet ist. Die Verbindungslinie zwischen der Position der Punktlichtquelle des Fixierlichts 20 und dem Krümmungszentrum der Kornea C_{c} stellt dabei die keratometrische Achse 300 dar, an deren Schnittpunkt mit der Außenfläche der Hornhaut 12a sich der korneale Vertex 22 befindet.

Ferner sind zur Erläuterung die Sichtlinie 400 des Auges 12 und der Kappa-Winkel 500, d.h. der Winkel zwischen der optischen Achse des Auges und der visuellen Achse 200 des Kontaktglases 16, eingezeichnet.

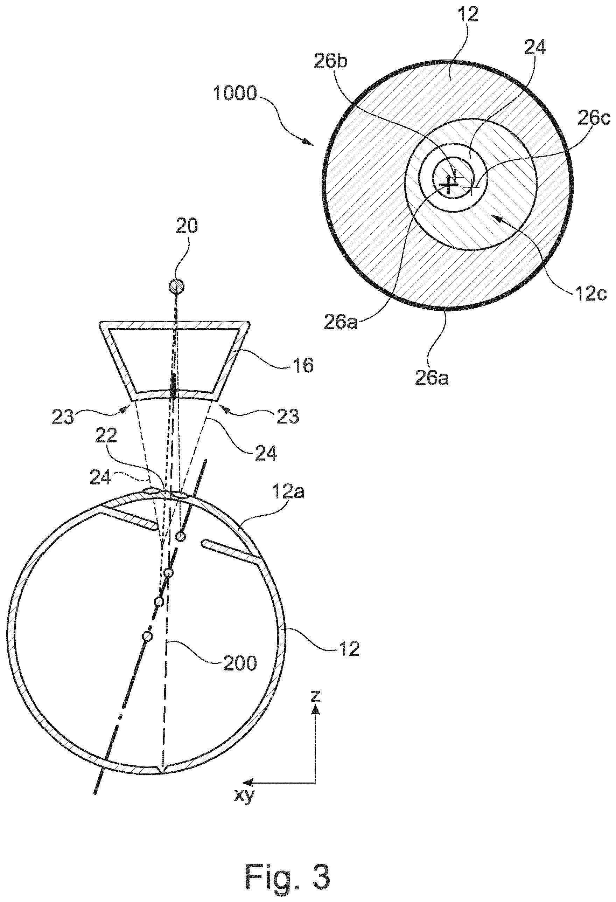

Im Folgenden wird anhand der Figuren 3 bis 5 ein Verfahren zur Zentrierung eines Kontaktglases 16 relativ zu einem Auge 12 gemäß einer optionalen Ausführungsform erläutert. Die Figuren 3 bis 5 zeigen dabei jeweils im linken unteren Bereich eine schematische Darstellung der relativen Anordnung des Kontaktglases zum Auge 12 und im rechten oberen Bereich eine Beispielhafte Darstellung 1000 einer Abbildung des Auges 12 mit überlagerten Markierungen.

In einem ersten Schritt (i) versichert sich der Arzt vor der refraktiven chirurgischen Behandlung des Auges 12, ob das Auge 12 problematische Irregularitäten auf der Oberfläche aufweist, welche der Behandlung entgegenstehen könnten. Sofern dies nicht der Fall ist, wird die Vorbereitung der Behandlung und die Zentrierung des Kontaktglases 12 fortgesetzt.

In einem zweiten Schritt (ii) erfolgt eine Befestigung des Kontaktglases 16 am Lasersystem 10 und eine Positionierung des befestigten Kontaktglases 16 über dem Auge 12 des Patienten 14 in einem z-Abstand von mehreren Zentimetern. Der Patient wird dazu angehalten, das Fixierlicht 20 mit dem Auge 12 zu fixieren.

In einem weiteren Schritt (iii) projiziert das Lasersystem 10 mittels einer Lichtquelle 23 ein Lichtmuster 24 auf das Auge 12, welche gemäß der optionalen Ausführungsform als ein um das Kontaktglaszentrum bzw. um die Zentralachse 200 konzentrischer Lichtring ausgebildet ist. Die Lichtquelle 23 ist gemäß einer optionalen Ausführungsform derart in und/oder am Kontaktglas 16 angeordnet, dass das von der Lichtquelle 23 emittierte Licht entlang des Rands des Kontaktglases 16 in Richtung des Auges 12 austritt und das ringförmige Lichtmuster 24 konzentrisch zur Zentralachse des Kontaktglases 16 bereitstellt. Das Lichtmuster 24 wird zumindest teilweise von der Hornhaut 12a reflektiert und ist demnach in der Abbildung der Lichtquelle 23 und des Auges 12 sichtbar und wird in der Darstellung 1000 der Abbildung des Auges 12 dargestellt. Gemäß der gezeigten Ausführungsform liegen die Ursprünge der Lichtstrahlen des Lichtmusters 24 konzentrisch um die Zentralachse 200 des Kontaktglases. Die an der Oberfläche des Auges reflektierten Lichtstrahlen sind dagegen konzentrisch um die keratometrische Achse angeordnet.

In der Darstellung 1000 der Abbildung des Auges 12, welche durch das Kontaktglas 16 hindurch aufgenommen wird, sind das Auge 12 und insbesondere die Pupille 12c erkennbar, sowie die Reflexion des Lichtmusters. Zudem werden in Überlagerung mit der Abbildung des Auges 12 mehrere virtuelle Markierungen 26 dargestellt, wobei die virtuelle Markierung 26a die Zentralachse 200 des Kontaktglases 16 und den Rand des Kontaktglases 16 kenntlich macht, die virtuelle Markierung 26b die Referenzmarkierung kenntlich macht, die aus der Abbildung des Lichtmusters 24 als auf der Zentralachse des Kontaktglases liegend abgeleitet ist, und die virtuelle Markierung 26c den mittels Bildauswertung aus der Abbildung ermittelten Mittelpunkt der Pupille 12c kenntlich macht.

Über die Darstellung 1000 kann der Arzt somit in einem Schritt (iv) den Kontaktglasrand, die Pupille und den Projektionsring erkennen.

Das System ermittelt dabei optional kontinuierlich und automatisiert den Mittelpunkt der Pupille, die Zentralachse des Kontaktglases 16 und den Mittelpunkt des Projektionsrings und zeigt die entsprechenden virtuellen Markierungen 26a, 26b, 26c in ständig aktualisierter Position an, sodass der Arzt diese mitverfolgen kann (Schritt (v)).

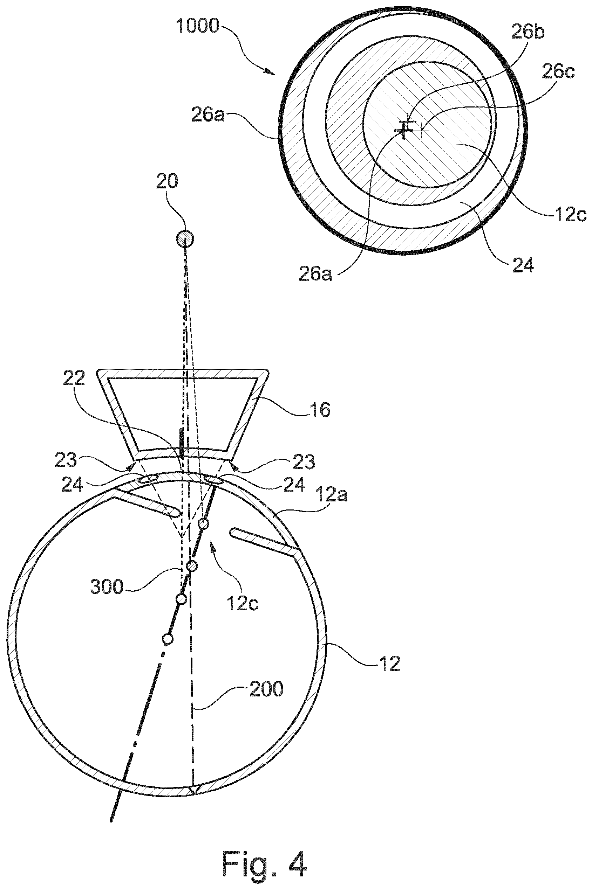

In einem weiteren Schritt (vi) kann, wie in Figur 4 dargestellt, der Arzt sodann den z-Abstand zwischen Kontaktglas 16 und Auge 12 verringern. Dabei vergrößert sich der Durchmesser des Projektionsrings auf dem Auge (siehe Darstellung 1000 in Figur 4). Der Arzt verringert den z-Abstand dabei soweit, bis der Projektionsring 24 größer ist als der Durchmesser der Pupille 12c aber kleiner als der Durchmesser des Kontaktglases 16 ist.

In einem weiteren Schritt (vii) pausiert der Arzt die Annäherung des Kontaktglases 16 an das Auge 12 und fordert den Patienten 14 nochmals dazu auf, das Fixierlicht 20 mit dem Auge 12 zu fixieren. Dann bringt der Arzt durch eine laterale Verschiebung des Patientenauges 12 relativ zum Kontaktglas 16 (oder umgekehrt) den Mittelpunkt des Kontaktglases 16. bzw. die Zentralachse 200 des Kontaktglases 16 mit dem Mittelpunkt des Lichtmusters 24 zur Deckung. In diesem Fall markiert das Zentrum des Lichtmusters 24 praktisch den Vertex 22 des Auges.

In einem weiteren Schritt (viii) veranlasst der Arzt das System dazu, dass dieses die mit Hilfe des Zentrums des Positionsrings 24 ermittelte Position des Vertex 22 abspeichert. Das System legt die ermittelte Position als Position des Vertex 22 fest und registriert diese Position automatisiert anhand eines in der dargestellten Abbildung des Auges 12 erkennbaren Merkmals des Auges 12. Gemäß dieser Ausführungsform wird vom Lasersystem 10 der Mittelpunkt der Pupille 12c als solches Merkmal herangezogen, da dieses mittels Bildauswertung zuverlässig erkennbar und bestimmbar ist. Gemäß anderen Ausführungsformen können jedoch alternativ oder zusätzlich auch andere Merkmale des Auges 12 herangezogen werden.

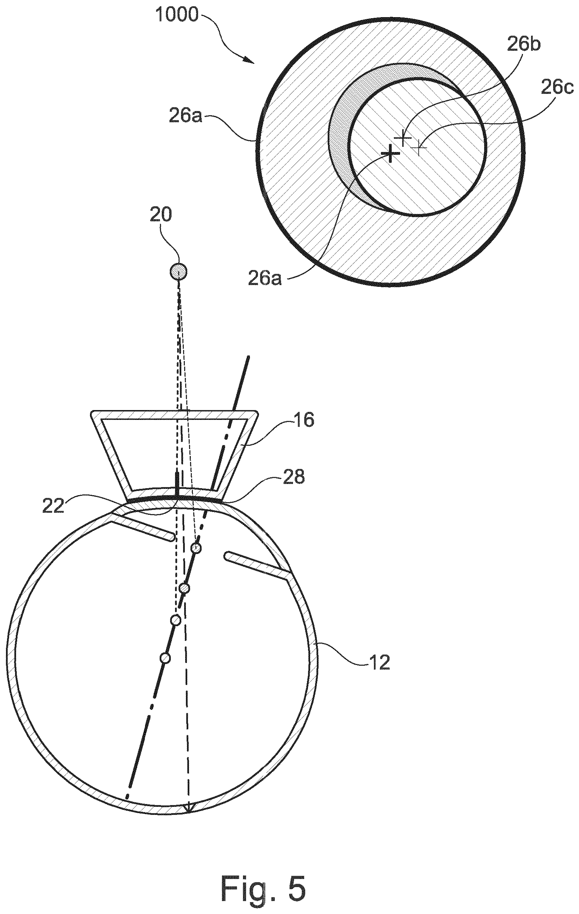

Anschließend setzt der Arzt in Schritt (ix) die Verringerung des z-Abstands zwischen dem Kontaktglas 16 und dem Auge 12 fort. Dabei wird nun vom Lasersystem 10 in der Darstellung 1000 die vom Lasersystem 10 gespeicherte und anhand des erkennbaren Merkmals registrierte Position des Vertex als virtuelle Markierung 26b fortgesetzt, auch wenn aufgrund des verringerten z-Abstands der Projektionsring 24 nicht mehr in der Abbildung sichtbar ist, wie in Figur 5 dargestellt. Dadurch bleibt die Position des Vertex 22 für den Arzt erkennbar und kann zur Orientierung verwendet werden. Der Arzt zentriert sodann das die Zentralachse 200 des Kontaktglases 16 auf die Position des Vertex 22 oder einen anderen von ihm vorgesehen Punkt des Auges 12.

Bei Kontakt des Kontaktglases 16 mit dem Auge 12 bildet sich ein Wassermeniskus 28 (Tränenfilm) zwischen dem Auge 12 und dem Kontaktglas 16, wie in Figur 5 dargestellt. Durch den Wassermeniskus wird die brechende Wirkung der Luft zwischen dem Kontaktglas 16 und dem Auge 12 vermieden. Die Hornhaut 12a wird durch den Kontakt mit dem Kontaktglas 16 deformiert bzw. applaniert, wobei sich der Krümmungsradius der Hornhaut 12a dem Krümmungsradius der Kontaktfläche 16a des Kontaktglases 16 anpasst. Das Lasersystem 10 berücksichtigt dabei die durch die Kontaktierung erfolgte Deformation der Hornhaut 12a und die Änderung des Abbildungsmaßstabs der Abbildung durch den Wassermeniskus und stellt dem Arzt die korrigierte Vertex-Position 22 dar.

Der Arzt kann sodann in einem weiteren Schritt (x) die Feinpositionierung des Kontaktglases 16 relativ zum Auge durchführen und sich dabei an den in der Darstellung angezeigten virtuellen Markierungen orientieren. Nach Abschluss der Feinpositionierung kann der Arzt den Abschluss bestätigen und das Ansaugen und Fixieren des Auges 12 an das Kontaktglas 16 herbeiführen.

Anschließend berechnet und protokolliert das Lasersystem 10 die Position des Kontaktglases 16 relativ zum Auge 12 und relativ zum erkennbaren Merkmal des Auges, wie etwa relativ zum Pupillenmittelpunkt, und optional den Durchmesser der Pupille und speichert diese zur Protokollierung des Vorgangs ab, sodass diese Information ggf. für eine nachträgliche Auswertung der refraktiven chirurgischen Behandlung bereitsteht.

### Bezugszeichenliste

- 10: refraktives chirurgisches Lasersystem
- 12: Auge
- 12a: Hornhaut
- 12b: Iris
- 12c: Pupille
- 14: Patient
- 15: Liege
- 16: Kontaktglas
- 17: Femtosekundenlaser
- 18: Anzeigeeinheit zur Darstellung einer Abbildung des Auges
- 19: Recheneinheit
- 20: Fixierlicht
- 22: Vertex bzw. Position des Vertex
- 23: Lichtquelle
- 24: Lichtmuster
- 26: virtuelle Markierung
- 26a: virtuelle Markierung der Zentralachse und des Randes Kontaktglases
- 26b: virtuelle Markierung des Mittelpunkts des Projektionsrings bzw. der projizierten optischen Markierung
- 26c: virtuelle Markierung des Mittelpunkts der Pupille
- 28: Wassermeniskus bzw. Tränenfilm

- 100: optische Achse des Auges
- 200: Zentralachse bzw. visuelle Achse des Kontaktglases
- 300: keratometrische Achse
- 400: Sichtlinie des Auges
- 500: Kappa-Winkel

- 1000: Darstellung der Abbildung des Auges

## Patentansprüche

1. Verfahren zur Zentrierung eines Kontaktglases (16) relativ zu einem Auge (12) eines Patienten, das Verfahren umfassend die Schritte:
a) Bereitstellen eines Fixierlichts (20) durch das Kontaktglas (16), sodass das Auge (12) des Patienten durch Fixieren auf das Fixierlicht (20) relativ zum Kontaktglas (16) ausgerichtet wird;
**dadurch gekennzeichnet, dass** das Verfahren ferner umfasst:
b) Erfassen einer Abbildung eines durch eine Lichtquelle (23) mit fester Ortsbeziehung relativ zum Kontaktglas (16) bereitgestellten Lichtmusters (24), wobei die Abbildung des Lichtmusters (24) über eine Reflexion an der Oberfläche des Auges (12) erfolgt;
c) Darstellen der Abbildung des Lichtmusters (24) über das Auge (12) durch das Kontaktglas (16) in Überlagerung mit virtuellen Markierungen (26), wobei eine erste Markierung (26a) der virtuellen Markierungen (26, 26a, 26b) die Zentralachse (200) des Kontaktglases (16) kenntlich macht und eine zweite Markierung (26b) der virtuellen Markierungen (26, 26a, 26b) eine Referenzmarkierung kenntlich macht, welche aus der Abbildung des Lichtmusters als auf der Zentralachse (200) des Kontaktglases (16) liegend abgeleitet ist;
d) Laterales Positionieren des Kontaktglases (16) relativ zum Auge (12) derart, dass ein Abstand zwischen der ersten Markierung (26a) und der zweiten Markierung (26b) minimiert wird;
e) Festlegen der Stelle des Auges (12), an welcher sich die erste Markierung (26a) befindet, wenn die erste Markierung und die zweite Markierung den minimierten Abstand zueinander einnehmen, als Position des Vertex (22) des Auges (12) und Registrieren der Position des Vertex (22) anhand eines in der Abbildung des Auges (12) erkennbaren Merkmals des Auges (12).

2. Verfahren gemäß Anspruch 1, ferner umfassend:
- Beleuchten des Auges mit einer Infrarot-Beleuchtung; und
- Erkennen der Pupille und optional des Mittelpunkts der Pupille des mit der Infrarot-Beleuchtung beleuchteten Auges.

3. Verfahren gemäß Anspruch 1 oder 2, wobei der Mittelpunkt der Pupille (12c) des Auges (12) als das in der Abbildung des Auges erkennbare Merkmal des Auges (12) herangezogen wird und wobei optional die virtuellen Markierungen (26) ferner eine dritte Markierung (26c) umfassen, welche den Mittelpunkt der Pupille (12c) kenntlich macht.

4. Verfahren gemäß einem der vorhergehenden Ansprüche, wobei Schritt c) ferner zumindest teilweise ein Anzeigen einer Topografie und/oder eines Wellenfrontbildes und/oder eines OCT-generierten Bildes des Auges (12) umfasst.

5. Verfahren gemäß Anspruch 4, ferner umfassend ein wahlweises Einblenden und/oder Ausblenden der Topografie und/oder des Wellenfrontbildes und/oder des OCT-generierten Bildes des Auges (12) und/oder ein Darstellen der Topografie und/oder eines Wellenfrontbildes und/oder eines OCT-generierten Bildes des Auges (12) als teiltransparentes Overlay.

6. Verfahren gemäß einem der vorhergehenden Ansprüche, ferner umfassend ein Überprüfen einer Überdeckung der Pupille durch eine optische Zone eines aus dem Auge herauszulösenden Lentikels, wobei das Überprüfen der Überdeckung optional unter Berücksichtigung einer Sicherheitsmarge erfolgt.

7. Verfahren gemäß Anspruch 3 oder einem der Ansprüche 4 bis 6, sofern auf Anspruch 3 rückbezogen, ferner umfassend ein Bestimmen eines Kappa-Winkels (500), d.h. des Winkels zwischen der optischen Achse des Auges und der visuellen Achse (200) des Kontaktglases (16), anhand der Position des Vertex (22) und des Mittelpunkts der Pupille (26c).

8. Verfahren gemäß Anspruch 7, ferner umfassend ein Ausgeben einer Warnung und/oder eines Hinweises an einen Benutzer, sofern der bestimmte Kappa-Winkel (500) um zumindest ein vorbestimmtes Maß von einer Vorgabe abweicht.

9. Verfahren gemäß einem der vorhergehenden Ansprüche, wobei das Darstellen der Abbildung des Lichtmusters (24) in Überlagerung mit virtuellen Markierungen (26) mittels einer Anzeigeeinheit erfolgt, und wobei die virtuellen Markierungen optional ausschließlich mittels der Anzeigeeinheit dargestellt werden, ohne auf das Auge (12) projiziert zu werden.

10. Verfahren gemäß Anspruch 9, wobei das Darstellen der Abbildung des Lichtmusters (24) in Überlagerung mit virtuellen Markierungen (26) mittels einer Anzeigeeinheit ferner ein Darstellen einer Abbildung des zu behandelnden Auges (12) umfasst.

11. Verfahren gemäß einem der Ansprüche 8 bis 10, wobei die Anzeigeeinheit ein Computerdisplay umfasst.

12. Verfahren gemäß einem der vorhergehenden Ansprüche, wobei das Erfassen der Abbildung des Lichtmusters (24) über die Reflexion an der Oberfläche des Auges (12) derart erfolgt, dass das Lichtmuster (24) und das Merkmal des Auges (12) in der Abbildung erkennbar sind, wobei die Abbildung mit einer Schärfentiefe von mindestens 10 mm, optional mindestens 15 mm, optional mindestens 20 mm und optional mindestens 30 mm erfolgt.

13. Verfahren gemäß einem der vorhergehenden Ansprüche, wobei bei der Durchführung Schritte a) bis e) das Kontaktglas (16) vom Auge (12) beabstandet ist und der Abstand des Kontaktglases (16) zum Auge (12) im Bereich von 1 mm bis 10 cm liegt.

14. Verfahren zur Vorbereitung einer refraktiven chirurgischen Behandlung eines Auges (12) mittels eines Lasersystems (10), das Verfahren umfassend ein Verfahren zur Zentrierung eines Kontaktglases (16) relativ zum Auge (12) gemäß einem der vorhergehenden Ansprüche und ein Ermitteln einer Überdeckung der Pupille (12c) des Auges (12) durch eine optische Zone eines bei der refraktiven chirurgischen Behandlung herauszulösenden Lentikels.

15. Recheneinheit (19), welche dazu eingerichtet ist, ein refraktives chirurgisches Lasersystem (10) zur Durchführung eines Verfahrens gemäß einem der vorhergehenden Ansprüche anzusteuern.

16. Refraktives chirurgisches Lasersystem (10), vorzugsweise ein Femtosekunden-Lasersystem mit einem Kontaktglas (16), wobei das Lasersystem (10) dazu eingerichtet ist,
a) ein Fixierlicht (20) durch das Kontaktglas (16) derart bereitzustellen, dass das Auge (12) eines Patienten durch Fixieren auf das Fixierlicht (20) relativ zum Kontaktglas (16) ausgerichtet wird;
**dadurch gekennzeichnet, dass** das Lasersystem ferner dazu eingerichtet ist:
b) eine Abbildung eines durch eine Lichtquelle (23) mit fester Ortsbeziehung relativ zum Kontaktglas (16) bereitgestellten Lichtmusters (24) zu erfassen, wobei die Abbildung des Lichtmusters (24) über eine Reflexion an der Oberfläche des Auges (12) erfolgt;
c) eine Abbildung des Lichtmusters (24) über die Reflexion an der Oberfläche des Auges (12) durch das Kontaktglas (16) in Überlagerung mit virtuellen Markierungen (26, 26a, 26b) darzustellen, wobei eine erste Markierung (26a) der virtuellen Markierungen (26, 26a, 26b) die Zentralachse (200) des Kontaktglases (16) kenntlich macht und eine zweite Markierung (26b) der virtuellen Markierungen (26, 26a, 26b) eine Referenzmarkierung kenntlich macht, welche aus der Abbildung des Lichtmusters (24) als auf der Zentralachse (200) des Kontaktglases (16) liegend abgeleitet ist;
d) das Kontaktglas (16) relativ zum Auge (12) derart lateral zu positionieren, dass ein Abstand zwischen der ersten Markierung (26a) und der zweiten Markierung (26b) minimiert wird;
e) die Stelle des Auges (12), an welcher sich die erste Markierung (26a) befindet, wenn die erste Markierung (26a) und die zweite Markierung (26b) den minimierten Abstand einnehmen, als Position des Vertex (22) des Auges (12) festzulegen und die Position des Vertex (22) anhand eines in der Abbildung des Auges (12) erkennbaren Merkmals des Auges (12) zu registrieren.

## Claims

1. Method for centring a contact glass (16) relative to an eye (12) of a patient, the method comprising the steps of:
a) providing a fixation light (20) by the contact glass (16) such that the patient's eye (12) is aligned relative to the contact glass (16) by fixation on the fixation light (20);
**characterized in that** the method further comprises:
b) capturing an image representation of a light pattern (24) provided by a light source (23) with a fixed spatial relationship relative to the contact glass (16), with the imaging of the light pattern (24) being implemented via a reflection off the surface of the eye (12);
c) displaying the image representation of the light pattern (24) via the eye (12) through the contact glass (16) with overlaid virtual markings (26), with a first marking (26a) of the virtual markings (26, 26a, 26b) identifying the central axis (200) of the contact glass (16) and a second marking (26b) of the virtual markings (26, 26a, 26b) identifying a reference marking which, from the image representation of the light pattern, is derived as being located on the central axis (200) of the contact glass (16);
d) laterally positioning the contact glass (16) relative to the eye (12) in such a way that a distance between the first marking (26a) and the second marking (26b) is minimized;
e) defining the location of the eye (12) at which the first marking (26a) is situated when the first marking and the second marking are at the minimized distance from one another as the position of the vertex (22) of the eye (12), and registering the position of the vertex (22) on the basis of a feature of the eye (12) that is recognizable in the image representation of the eye (12).

2. Method according to Claim 1, further comprising:
- illuminating the eye with infrared illumination; and
- recognizing the pupil and optionally the centre of the pupil of the eye illuminated with the infrared illumination.

3. Method according to Claim 1 or 2, wherein the centre of the pupil (12c) of the eye (12) is used as the feature of the eye (12) that is recognizable in the image representation of the eye and wherein optionally the virtual markings (26) further comprise a third marking (26c) which identifies the centre of the pupil (12c).

4. Method according to any of the preceding claims, wherein step c) further at least partially comprises a display of a topography and/or wavefront image and/or OCT-generated image of the eye (12).

5. Method according to Claim 4, further comprising optionally showing and/or hiding the topography and/or the wavefront image and/or the OCT-generated image of the eye (12) and/or representing the topography and/or a wavefront image and/or an OCT-generated image of the eye (12) as a partially transparent overlay.

6. Method according to any of the preceding claims, further comprising checking an overlap of the pupil with an optical zone of a lenticule to be extracted from the eye, wherein the overlap is optionally checked taking account of a safety margin.

7. Method according to Claim 3 or any of Claims 4 to 6, if referred back to Claim 3, further comprising determining a kappa angle (500), i.e. the angle between the optical axis of the eye and the visual axis (200) of the contact glass (16), on the basis of the position of the vertex (22) and the centre of the pupil (26c).

8. Method according to Claim 7, further comprising outputting a warning and/or a message to a user if the determined kappa angle (500) deviates from a specification by at least a predetermined amount.

9. Method according to any of the preceding claims, wherein the image representation of the light pattern (24) with overlaid virtual markings (26) is displayed by means of a display unit, and wherein the virtual markings are optionally displayed exclusively by means of the display unit without being projected onto the eye (12).

10. Method according to Claim 9, wherein displaying the image representation of the light pattern (24) with overlaid virtual markings (26) by means of a display unit further comprises displaying an image representation of the eye (12) to be treated.

11. Method according to any of Claims 8 to 10, wherein the display unit comprises a computer display.

12. Method according to any of the preceding claims, wherein the capturing of the image representation of the light pattern (24) via the reflection off the surface of the eye (12) is implemented in such a way that the light pattern (24) and the feature of the eye (12) are recognizable in the image representation, wherein the imaging is effected with a depth of field of at least 10 mm, optionally at least 15 mm, optionally at least 20 mm and optionally at least 30 mm.

13. Method according to any of the preceding claims, wherein the contact glass (16) is spaced apart from the eye (12) when steps a) to e) are implemented and the distance of the contact glass (16) from the eye (12) is in the range from 1 mm to 10 cm.

14. Method for preparing a refractive surgical treatment of an eye (12) by means of a laser system (10), the method comprising a method for centring a contact glass (16) relative to the eye (12) according to any of the preceding claims and an ascertainment of an overlap of the pupil (12c) of the eye (12) with an optical zone of a lenticule to be extracted during refractive surgery.

15. Computing unit (19) configured to control a refractive surgical laser system (10) for implementing a method according to any of the preceding claims.

16. Refractive surgical laser system (10), preferably a femtosecond laser system with a contact glass (16), the laser system (10) being configured to
a) provide a fixation light (20) by the contact glass (16) in such a way that the patient's eye (12) is aligned relative to the contact glass (16) by fixation on the fixation light (20);
**characterized in that** the laser system is furthermore configured to:
b) capture an image representation of a light pattern (24) provided by a light source (23) with a fixed spatial relationship relative to the contact glass (16), with the imaging of the light pattern (24) being implemented via a reflection off the surface of the eye (12);
c) display an image representation of the light pattern (24) via the reflection off the surface of the eye (12) through the contact glass (16) with overlaid virtual markings (26, 26a, 26b), with a first marking (26a) of the virtual markings (26, 26a, 26b) identifying the central axis (200) of the contact glass (16) and a second marking (26b) of the virtual markings (26, 26a, 26b) identifying a reference marking which, from the image representation of the light pattern (24), is derived as being located on the central axis (200) of the contact glass (16);
d) laterally position the contact glass (16) relative to the eye (12) in such a way that a distance between the first marking (26a) and the second marking (26b) is minimized;
e) define the location of the eye (12) at which the first marking (26a) is situated when the first marking (26a) and the second marking (26b) are at the minimized distance as the position of the vertex (22) of the eye (12), and register the position of the vertex (22) on the basis of a feature of the eye (12) that is recognizable in the image representation of the eye (12).

## Revendications

1. Procédé de centrage d'une lentille de contact (16) par rapport à l'œil (12) d'un patient, le procédé comprenant les étapes suivantes :
a) la fourniture d'une lumière de fixation (20) à travers la lentille de contact (16) de sorte que l'œil (12) du patient est aligné par rapport à la lentille de contact (16) par fixation de la lumière de fixation (20) ;
**caractérisé en ce que** le procédé comprend en outre :
b) la détection d'une image d'un motif lumineux (24) fourni par une source lumineuse (23) ayant une relation spatiale fixe par rapport à la lentille de contact (16), l'image du motif lumineux (24) étant formée par le biais d'une réflexion à la surface de l'œil (12) ;
c) la représentation de l'image du motif lumineux (24) sur l'œil (12) au moyen de la lentille de contact (16) en superposition avec des marquages virtuels (26), un premier marquage (26a) des marquages virtuels (26, 26a, 26b) identifiant l'axe central (200) de la lentille de contact (16) et un deuxième marquage (26b) des marquages virtuels (26, 26a, 26b) identifiant un marquage de référence, qui est issu de l'image du motif lumineux comme étant situé sur l'axe central (200) de la lentille de contact (16) ;
d) le positionnement latéral de la lentille de contact (16) par rapport à l'œil (12) de telle sorte qu'une distance entre le premier marquage (26a) et le deuxième marquage (26b) soit minimisée ;
e) l'établissement de la position de l'œil (12) à laquelle le premier marquage (26a) est situé lorsque le premier marquage et le deuxième marquage adoptent la distance minimale l'un de l'autre en tant que position du sommet (22) de l'œil (12), et l'enregistrement de la position du sommet (22) à l'aide d'une caractéristique de l'œil (12) reconnaissable dans l'image de l'œil (12).

2. Procédé selon la revendication 1, comprenant en outre :
- l'éclairage de l'œil par un éclairement infrarouge ; et
- la détection de la pupille et facultativement du centre de la pupille de l'œil éclairé par l'éclairement infrarouge.

3. Procédé selon la revendication 1 ou 2, dans lequel le centre de la pupille (12c) de l'œil (12) est utilisé en tant que caractéristique de l'œil (12) reconnaissable dans l'image de l'œil, et les marquages virtuels (26) comprenant en outre facultativement un troisième marquage (26c) qui identifie le centre de la pupille (12c).

4. Procédé selon l'une des revendications précédentes, dans lequel l'étape c) comprend en outre, au moins en partie, un affichage d'une topographie et/ou d'une image de front d'onde et/ou d'une image générée par OCT de l'œil (12).

5. Procédé selon la revendication 4, comprenant en outre une incrustation et/ou un masquage sélectifs de la topographie et/ou de l'image de front d'onde et/ou de l'image générée par OCT de l'œil (12) et/ou une représentation de la topographie et/ou d'une image de front d'onde et/ou d'une image générée par OCT de l'œil (12) sous forme de calque partiellement transparent.

6. Procédé selon l'une des revendications précédentes, comprenant en outre la vérification d'un recouvrement de la pupille par une zone optique d'une lenticule à détacher de l'œil, la vérification du recouvrement étant facultativement effectuée en tenant compte d'une marge de sécurité.

7. Procédé selon la revendication 3 ou l'une des revendications 4 à 6, lorsqu'elles dépendent de la revendication 3, comprenant en outre la détermination d'un angle kappa (500), c'est-à-dire de l'angle entre l'axe optique de l'œil et l'axe visuel (200) de la lentille de contact (16), à partir de la position du sommet (22) et du centre de la pupille (26c).

8. Procédé selon la revendication 7, comprenant en outre l'émission d'un avertissement et/ou d'une indication à destination d'un utilisateur si l'angle kappa déterminé (500) s'écarte d'une valeur prédéfinie d'au moins une mesure prédéterminée.

9. Procédé selon l'une des revendications précédentes, dans lequel la représentation de l'image du motif lumineux (24) en superposition avec des marquages virtuels (26) est réalisée au moyen d'une unité d'affichage, et dans lequel les marquages virtuels sont facultativement représentés uniquement par l'unité d'affichage, sans être projetés sur l'œil (12).

10. Procédé selon la revendication 9, dans lequel la présentation de l'image du motif lumineux (24) en superposition avec des marquages virtuels (26) au moyen d'une unité d'affichage comprend en outre une représentation de l'image de l'œil (12) à traiter.

11. Procédé selon l'une des revendications 8 à 10, dans lequel l'unité d'affichage comprend un dispositif d'affichage d'ordinateur.

12. Procédé selon l'une des revendications précédentes, dans lequel la détection de l'image du motif lumineux (24) par réflexion à la surface de l'œil (12) est effectuée de manière à ce que le motif lumineux (24) et la caractéristique de l'œil (12) soient reconnaissables dans l'image, l'image étant formée avec une profondeur de champ d'au moins 10 mm, facultativement d'au moins 15 mm, facultativement d'au moins 20 mm et facultativement d'au moins 30 mm.

13. Procédé selon l'une des revendications précédentes, dans lequel, lors de le mise en œuvre des étapes a) à e), la lentille de contact (16) est espacée de l'œil (12) et la distance entre la lentille de contact (16) et l'œil (12) se situe dans la plage de 1 mm à 10 cm.

14. Procédé de préparation d'un traitement chirurgical réfractif d'un œil (12) au moyen d'un système laser (10), le procédé comprenant un procédé de centrage d'une lentille de contact (16) par rapport à l'œil (12) selon l'une des revendications précédentes et une détermination d'un recouvrement de la pupille (12c) de l'œil (12) par une zone optique d'une lenticule à détacher lors du traitement chirurgical réfractif.

15. Unité de calcul (19), conçue pour commander un système laser chirurgical réfractif (10) afin de mettre en œuvre un procédé selon l'une des revendications précédentes.

16. Système laser chirurgical réfractif (10), de préférence un système laser femtoseconde comportant une lentille de contact (16), le système laser (10) étant conçu pour
a) fournir une lumière de fixation (20) à travers la lentille de contact (16) de sorte que l'œil (12) d'un patient est aligné par rapport à la lentille de contact (16) par fixation de la lumière de fixation (20) ;
**caractérisé en ce que** le système laser est en outre conçu pour :
b) détecter une image d'un motif lumineux (24) fourni par une source lumineuse (23) ayant une relation spatiale fixe par rapport à la lentille de contact (16), l'image du motif lumineux (24) étant formée par le biais d'une réflexion à la surface de l'œil (12) ;
c) représenter une image du motif lumineux (24) sur la réflexion à la surface de l'œil (12) au moyen de la lentille de contact (16) en superposition avec des marquages virtuels (26, 26a, 26b), un premier marquage (26a) des marquages virtuels (26, 26a, 26b) identifiant l'axe central (200) de la lentille de contact (16) et un deuxième marquage (26b) des marquages virtuels (26, 26a, 26b) identifiant un marquage de référence, qui est issu de l'image du motif lumineux (24) comme étant situé sur l'axe central (200) de la lentille de contact (16) ;
d) positionner latéralement la lentille de contact (16) par rapport à l'œil (12) de telle sorte qu'une distance entre le premier marquage (26a) et le deuxième marquage (26b) soit minimisée ;
e) établir la position de l'œil (12) à laquelle le premier marquage (26a) est situé lorsque le premier marquage (26a) et le deuxième marquage (26b) adoptent la distance minimale en tant que position du sommet (22) de l'œil (12), et enregistrer la position du sommet (22) à l'aide d'une caractéristique de l'œil (12) reconnaissable dans l'image de l'œil (12).
